# EUROPÄISCHE PATENTSCHRIFT

(11) **EP 0 315 730 B1**
(45) Veröffentlichungstag und Bekanntmachung des Hinweises auf die Patenterteilung: **08.06.1994**
(21) Anmeldenummer: 88107297.9
(22) Anmeldetag: 06.05.1988
(51) Int. Cl.: A61B 17/39, A61F 7/12

(54) **Vorrichtung zum Erweiten und/oder Eröffnen von Blutgefässen**
Device for dilating and/or opening blood vessels
Dispositif pour la dilatation et/ou l'ouverture de vaisseaux sanguins

(30) Priorität: 12.11.1987 DE 3738429
(43) Veröffentlichungstag der Anmeldung: 17.05.1989
(73) Patentinhaber: Osypka, Peter, Dr. Ing., 79639 Grenzach-Wyhlen (DE)
(72) Erfinder: Osypka, Peter, Dr. Ing., D-7889 Grenzach-Wyhlen (DE); Hombach, Vinzenz, Prof. Dr. med., D-7900 Ulm (DE)
(74) Vertreter: Schmitt, Hans, Dipl.-Ing.

(56) Entgegenhaltungen:
- CH-A- 566 790
- DE-A- 3 406 294
- GB-A- 988 130
- US-A- 1 731 627
- US-A- 2 056 377
- US-A- 3 460 539
- US-A- 3 858 586
- US-A- 4 672 962
- US-A- 4 682 596

## Beschreibung

Die Erfindung betrifft eine Vorrichtung zum Erweitern und/oder Eröffnen von - krankhaft verengten - Blutgefäßen mit Hilfe von Wärme gemäß dem Oberbegriff des Patentanspruches 1.

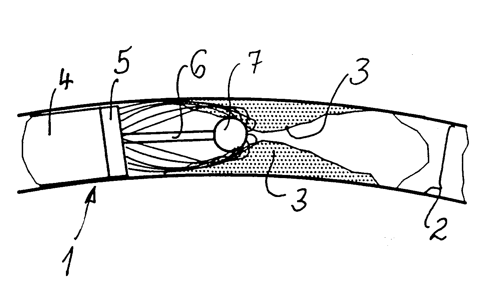

Eine derartige Vorrichtung ist aus der US-A-4 682 596 und dabei insbesondere den Figuren 10 und 11 bekannt. Die Elektrodenanordnung hat dabei zwei in einem festen Abstand zueinander angeordnete Pole. Ein im Querschnitt verminderter Führungsdraht besteht aus Metall und hat einen dicken isolierenden Überzug. Der Führungsdraht ist also beim Vorschieben dieser Vorrichtung in einem verengten Gefäß das Teil, welches mit der Verengung zuerst in Berührung kommt, bevor die in ihrem Querschnitt erheblich dickere Elektrodenanordnung mit ihren Polen in den Bereich der zu beseitigenden oder zu erweiternden Engstelle gelangt. Durch die dadurch auftretende mechanische Aufweitung kann das befallene Gefäß überbeansprucht werden.

Der Erfindung liegt die Aufgabe zugrunde, eine Vorrichtung der eingangs erwähnten Art zu schaffen, welche einfach im Aufbau ist, eine gezielte und auf den jeweiligen Anwendungsfall dosierbare lokale Aufbringung der Wärme erlaubt, ohne daß die eigentliche Gefäßwand belastet werden muß.

Diese Aufgabe wird bei einer Vorrichtung gem. dem Oberbegriff des Patentanspruches 1 mit Hilfe der Merkmale und Maßnahmen des kennzeichnenden Teiles des Patentanspruches 1 gelöst.

Die Elektrodenanordnung der erfindungsgemäßen Vorrichtung kann also mit dem Gegenpol an und in die Gefäßverengung geschoben werden, wo durch unmittelbar aufgebrachte Hochfrequenzenergie auf einfache Weise die jeweils erforderliche Wärme erzeugt werden kann, um die Gefäßverengung durch eine Hochfrequenz-Tomie ganz oder wenigstens teilweise zu beseitigen. Dabei ist vorteilhaft, daß die Gefäßwand selbst nicht unbedingt einem Innendruck ausgesetzt werden muß. Die Elektrodenanordnungen in Form von Kathetern sind vielfältig, insbesondere auch bei der Herz-Therapie bekannt, so daß die Technik dafür ohne weiteres zur Verfügung steht.

Durch die axiale relative Verstellbarkeit von Pol und Gegenpol kann im Arbeitsfortschritt der Wärmeanwendung immer abwechselnd zunächst die Führung in die Engstelle tiefer eingeschoben werden, wonach dann der nachführbare Pol dieser Führung entlang nachfolgen kann. Durch dieses abwechselnde Vorschieben ist ein schonendes und präzises Abarbeiten der Verengung möglich, wobei die Gefahr größerer Ablösungen praktisch ausgeschlossen werden kann. Dabei können die Vorteile einer bipolaren Koagulation in Anwendung kommen, das heißt die Hochfrequenzenergie kann ganz gezielt an dem Einsatzort aufgebracht und angewendet werden und es besteht nicht die Gefahr, daß an einer am Körper anlegbaren Elektrode Verbrennungen auftreten.

Um das Vorschieben der Führung mit dem Gegenpol möglichst schonend durchführen zu können, kann die in Vorschubrichtung vorderste Stirnseite der relativ zu dem Pol verschiebbaren Führung abgerundet, ggf. kugelig verdickt sein. Vor allem kann der Gegenpol als kugelige Verdickung des vorderen Endes der Führung ausgebildet sein.

Die relativ zu dem Pol bewegbare und verstellbare Führung kann durch die gesamte elektrische Zuleitung bis zu deren Anschlußende verlaufen und an diesem Anschlußende überstehen und gegebenenfalls einen Stellgriff aufweisen. Somit kann der Operateur von dem Eintritt der Elektrodenanordnung aus in den Patientenkörper die erforderlichen wechselweisen Vorschubbewegungen der Führung mit dem Gegenpol und des nachfolgenden Poles gut durchführen. Dabei kann die Gesamtoberfläche des vorderen, die Führung mitbildenden Gegenpoles kleiner als die Oberfläche des nachfolgenden Poles sein. Dadurch wird sichergestellt, daß die Hochfrequenzenergie von dem vorderen kleineren Gegenpol aus zu dem nachfogenden Pol geleitet wird. Entsprechend gezielt kann der vordere Gegenpol allmählich in die Gefäßverengung eingeführt werden und diese dabei erweitern oder beseitigen.

Die Führung und/oder der von ihr getragene Gegenpol kann dabei relativ zu dem anderen Pol um etwa 1 bis 2 cm verstellbar sein. Innerhalb eines solchen sich ändernden Abstandes kann die Hochfrequenzenergie gut übertragen werden.

Vor allem bei Kombination der vorbeschriebenen Merkmale und Maßnahmen ergibt sich eine Vorrichtung mit einer Elektrodenanordnung, mit welcher Gefäßverengungen durch Hochfrequenz-Tomie auf einfache Weise gezielt mit Wärme behandelt werden können, wobei die Vorrichtung in vorteilhafter Weise sehr preiswert sein kann.

Eine weitere Ausgestaltung kann vorsehen, daß der Pol oder Gegenpol einen eingebauten Temperaturfühler aufweist. Dadurch kann der Hochfrequenzgenerator derart geregelt werden, daß er jeweils nur soviel Energie abgibt, daß die Temperatur an der Elektrodenspitze konstant gehalten wird oder nach einem vorgewählten Programm eventuell allmählich gesteigert und dann wieder zurückgenommen wird.

Es sei noch erwähnt, daß der Führungsdraht den indifferenten Pol einer bipolaren Katheteranordnung aufweisen kann. Auch dadurch kann eine indifferente Körperelektrode, an der es unter Umständen zu Verbrennungen kommen kann, vermieden werden.

Zum zeitweiligen Verankern der Elektrodenanordnung in einem Gefäß, einer Ader oder dergleichen kann an dem den Führungsdraht oder dergleichen aufweisenden Katheter wenigstens ein Verankerungsballon vorgesehen sein. Damit kann eine Festlegung in Gebrauchsstellung erreicht werden, während der Führungsdraht, der in vorteilhafter Weise selbst eine elektrische Zuleitung ist, verschoben wird.

Nachstehend ist ein Ausführungsbeispiel der Erfindung anhand der Zeichnung näher beschrieben. Es zeigt in schematisierter Darstellung
die einzige Figur
das Arbeitsende einer als Elektrodenanordnung ausgebildeten Vorrichtung zum Erweitern oder Eröffnen von Blutgefäßen in bipolarer Ausführung, wobei ein Führungsdraht mit einem Gegenpol in die Engstelle geschoben ist.

Eine im ganzen mit 1 bezeichnete, nur bezüglich ihres Arbeitsendes dargestellte Vorrichtung zum Erweitern und/oder Eröffnen von krankhaft vorschlossenen Blutgefäßen 2 mit Hilfe von Wärme ist durch zu der zu erweiternden Engstelle 3 hin führende Gefäße einführbar und hat einen noch zu beschreibenden Arbeitskopf, mit welchem Wärme zugeführt werden kann, um die Gefäßverengung 3 ganz oder teilweise damit zu beseitigen.

Die Vorrichtung 1 ist als eine mit elektrischer Zuleitung 4 versehene Elektrodenanordnung ausgebildet, die an einen nicht näher dargestellten Hochfrequenzgenerator anschließbar ist. Dabei ist eine weitere Elektrode vorgesehen, um von dem Pol 5 im Inneren des Gefäßes am Ende der elektrischen Zuleitung 4 die Hochfrequenzenergie auf die Gefäßverengung 3 zu übertragen.

Das vordere Ende einer noch zu beschreibenden Führung 6 und eine an dem Ende dieser Führung 6 vorgesehene kugelige Verdickung ist als Gegenpol 7 zu dem nachführbaren Pol 5, also die Führung 6 als weitere Elektrode ausgebildet und gegenüber dem Pol 5 isoliert. Die Elektrodenanordnung ist also bipolar. Somit kann die Hochfrequenzenergie von dem Gegenpol 7 zu dem Pol 5 übergehen und gezielt und genau zur Beseitigung der Verengung 3 eingesetzt werden, wobei in vorteilhafter Weise immer gerade der Bereich, an welchem der Gegenpol 7 in die Gefäßverengung 3 eindringt, durch die auftretende Hochfrequenz-Tomie beseitigt und erweitert wird.

Dabei ist vorgesehen, daß aus der in Erstreckungsrichtung der Elektrodenanordnung und des Gefäßes 2 orientierten Oberfläche des Poles 5 gegenüber diesem die schon erwähnte, im Querschnitt verminderte Führung 6, im Ausführungsbeispiel ein Führungsdraht vorsteht. Die Führung 6 ist relativ zu dem Pol 5 axial verstellbar und vorzugsweise während des Vorschubes des Poles 5 feststehend und bei stationärem Pol 5 vorschiebbar. Somit kann also abwechselnd die Führung 6 und der Pol 5 weiter und weiter in die Gefäßverengung 3 hineingeschoben werden, wobei der Operateur dies vorsichtig und gezielt gemäß dem Fortschritt durchführen kann, in welchem durch die Hochfrequenz-Tomie die Verengung 3 allmählich beseitigt wird.

Die relativ zu dem Pol 5 bewegbare und verstellbare Führung 6 verläuft durch die gesamte elektrische Zuleitung 4 bis zu deren in den Zeichnungen nicht dargestellten Anschlußende, steht an diesem Anschlußende über und weist dort gegebenenfalls einen Stellgriff auf, damit der Benutzer die vorbeschriebenen abwechselnden Vorschubbewegungen der Führung 6 und des Poles 5 bequem und wohl dosiert durchführen kann.

Es sei noch erwähnt, daß die Gesamtoberfläche des vorderen, die Führung 6 mitbildenden Gegenpoles 7 kleiner als die Oberfläche des nachfolgenden Poles 5 ist, damit die Hochfrequenzenergie von diesem Gegenpol 7 ausschließlich zum Pol 5 fließt.

Die Führung 6 und/oder der von ihr getragene Gegenpol 7 können relativ zu dem Pol 5 um etwa 1 bis 2 cm verstellbar sein. Dies genügt, um abwechselnd die Führung 6 in Position zu bringen und dann den Pol 5 nachzuführen, um die eigentliche Beseitigung der Verengung 3 zu bewirken.

Insgesamt ergibt sich eine technisch und vom apparativen Aufwand relativ einfache Vorrichtung, mit der mit Hilfe von Hochfrequenz-Tomie Gefäßverengungen gezielt und dennoch schonend durch Wärmeeinwirkung beseitigt oder vermindert werden können, wobei diese Methode natürlich auch noch mit weiteren Methoden zur Behandlung solcher Gefäßverengungen kombiniert sein könnte, insbesondere mit mechanischen Methoden zur Verdrängung im Bereich der Verengung 3.

## Patentansprüche

1. Vorrichtung (1) zum Erweitern und/oder Eröffnen von Blutgefäßen mit Hilfe von Wärme, wobei die Vorrichtung (1) durch zu der Engstelle hin führende Gefäße einführbar ist und einen Arbeitskopf zum Aufweiten der Gefäßverengung oder des Gefäßverschlusses hat, als eine eine elektrische Zuleitung (4) und eine weitere Elektrode aufweisende bipolare Elektrodenanordnung mit einem an ihrem Arbeitsende befindlichen Pol (5) und einem demgegenüber isolierten Gegenpol (7) ausgebildet ist und an einen Hochfrequenzgenerator anschließbar ist, wobei aus der Oberfläche des Poles (5) eine gegenüber diesem im Querschnitt verminderte und in Erstreckungsrichtung des Gefäßes (2) orientierte Führung (6), insbesondere ein Führungsdraht, vorsteht, **dadurch gekennzeichnet**, daß die Führung (6) als weitere Elektrode und ihr vorderes Ende als der Gegenpol (7) zu dem nachführbaren Pol (5) ausgebildet sind und daß die den Gegenpol (7) aufweisende Führung (6) relativ zu dem Pol (5) axial verstellbar ist.

2. Vorrichtung nach Anspruch 1, dadurch gekennzeichnet, daß die in Vorschubrichtung vorderste Stirnseite der relativ zu dem Pol (5) verschiebbaren Führung (6) abgerundet, gegebenenfalls kugelig verdickt ist.

3. Vorrichtung nach Anspruch 1 oder 2, dadurch gekennzeichnet, daß der Gegenpol (7) als kugelige Verdickung des vorderen Endes der Führung (6) ausgebildet ist.

4. Vorrichtung nach einem der Ansprüche 1 bis 3, dadurch gekennzeichnet, daß die relativ zu dem Pol (5) bewegbare und verstellbare Führung (6) durch die gesamte elektrische Zuleitung (4) bis zu deren Anschlußende verläuft und an diesem Anschlußende übersteht und gegebenenfalls einen Stellgriff aufweist.

5. Vorrichtung nach einem der Ansprüche 1 bis 4, dadurch gekennzeichnet, daß die Gesamtoberfläche des vorderen, die Führung (6) mitbildenden Gegenpoles (7) kleiner als die Oberfläche des nachfolgenden Poles (5) ist.

6. Vorrichtung nach einem der Ansprüche 1 bis 5, dadurch gekennzeichnet, daß die Führung (6) und/oder der von ihr getragene Gegenpol (7) relativ zu dem anderen Pol (5) um etwa 1 bis 2 cm verstellbar ist.

7. Vorrichtung nach einem der Ansprüche 1 bis 6, dadurch gekennzeichnet, daß der Pol (5) oder Gegenpol (7) einen eingebauten Temperaturfühler aufweist.

8. Vorrichtung nach einem der vorstehenden Ansprüche, dadurch gekennzeichnet, daß der Führungsdraht (6) den indifferenten Pol einer bipolaren Katheteranordnung aufweist.

9. Vorrichtung nach einem der Ansprüche 1 bis 8, dadurch gekennzeichnet, daß zum zeitweiligen Verankern der Elektrodenanordnung in einem Gefäß (2), z.B. einer Ader, an dem den Führungsdraht (6) aufweisenden Katheter wenigstens ein Verankerungsballon vorgesehen ist.

## Claims

1. A device (1) for dilating and/or opening blood vessels with the aid of heat, wherein the device (1) is adapted to be inserted through vessels leading to the narrow and has a working head for dilating the vasoconstriction or vascular occlusion, wherein said device takes the form of a bipolar electrode arrangement having an electric lead (4) and a further electrode and additionally having a pole (5) situated at the working end thereof and an antipole (7) insulated from said pole and is connectable to a high-frequency generator, wherein projecting from the surface of the pole (5) is a guide (6), particularly a guidewire, which is of reduced cross-section compared to said pole and is oriented in the direction in which the vessel (2) extends, **characterized in that** the guide (6) takes the form of the further electrode and the front end thereof takes the form of the antipole (7) to the follow-up pole (5), and that the guide (6) having the antipole (7) is axially adjustable relative to the pole (5).

2. A device as claimed in claim 1, characterized in that the guide (6) movable relative to the pole (5) has its foremost end in the direction of feed rounded, possibly spherically thickened.

3. A device as claimed in claim 1 or claim 2, characterized in that the antipole (7) takes the form of the spherical thickening of the front end of the guide (6).

4. A device as claimed in any one of claims 1 to 3, characterized in that the guide (6) movable and adjustable relative to the pole (5) runs throughout the electric lead (4) to the connecting end thereof and projects at said connecting end and may have an adjusting grip.

5. A device as claimed in any one of claims 1 to 4, characterized in that the overall surface of the front antipole (7) jointly constituting the guide (6) is smaller than the surface of the following pole (5).

6. A device as claimed in any one of claims 1 to 5, characterized in that the guide (6) and/or the antipole (7) carried thereby is movable about 1 to 2 cms relative to the other pole (5).

7. A device as claimed in any one of claims 1 to 6, characterized in that the pole (5) or antipole (7) has an incorporated temperature sensor.

8. A device as claimed in any one of the preceding claims, characterized in that the guidewire (6) has the indifferent pole of a bipolar catheter arrangement.

9. A device as claimed in any one of claims 1 to 8, characterized in that at least one anchoring balloon is provided on the catheter having the guidewire (6) and serves for temporarily anchoring the electrode arrangement in a vessel (2), e.g. a blood vessel.

## Revendications

1. Dispositif (1) pour dilater et/ou ouvrir les vaisseaux sanguins à l'aide de chaleur, dispositif qui peut être introduit à travers des vaisseaux menant jusqu'au rétrécissement et possède une tête de travail, destinée à la dilatation du rétrécissement ou de l'occlusion du vaisseau, sous la forme d'un dispositif d'électrode bipolaire, présentant une connexion électrique (4) et une électrode supplémentaire, comportant un pôle (5) situé à son extrémité de travail et un pôle opposé (7) isolé par rapport à lui, et pouvant être raccordé à un générateur de haute fréquence, un guide (6) de section réduite, en particulier un fil de guidage, dépassant de la surface du pôle (5) et étant orienté, par rapport à celui-ci, dans la direction d'extension du vaisseau (2),caractérisé en ce que le guide (6) est réalisé en tant qu'électrode supplémentaire et son extrémité avant est réalisée comme le pôle opposé (7) par rapport au pôle (5), pouvant être guidé à sa suite, et que le guide (6), présentant le pôle opposé (7), est déplaçable axialement par rapport au pôle (5).

2. Dispositif selon la revendication 1, caractérisé en ce que l'extrémité avant, dans la direction d'avancement, du guide (6) déplaçable par rapport au pôle (5), est arrondie, éventuellement épaissie sphériquement.

3. Dispositif selon la revendication 1 ou 2, caractérisé en ce que le pôle opposé (7) est réalisé comme un épaississement sphérique de l'extrémité avant du guide (6).

4. Dispositif selon une des revendications 1 à 3, caractérisé en ce que le guide (6), qui est mobile et déplaçable par rapport au pôle (5), s'étend à travers toute la connexion électrique (4), jusqu'à son extrémité de raccordement, dépasse de cette extrémité de raccordement et présente éventuellement un élément de préhension pour le déplacement.

5. Dispositif selon une des revendications 1 à 4, caractérisé en ce que la surface totale du pôle opposé (7), situé à l'avant et contribuant a la formation du guide (6), est plus petite que la surface du pôle (5) venant à la suite.

6. Dispositif selon une des revendications 1 à 5, caractérisé en ce que le guide (6) et/ou le pôle opposé (7), porté par lui, est déplaçable d'environ 1 à 2 cm par rapport à l'autre pôle (5).

7. Dispositif selon une des revendications 1 à 6, caractérisé en ce que le pôle (5) ou le pôle opposé (7) comporte un capteur de température incorporé.

8. Dispositif selon une des revendications précédentes, caractérisé en ce que le fil de guidage (6) présente le pôle neutre d'un dispositif de cathéter bipolaire.

9. Dispositif selon une des revendications 1 à 8, caractérisé en ce que, pour l'ancrage temporaire du dispositif d'électrode dans un vaisseau (2), une artère par exemple, au moins un ballonnet d'ancrage est prévu sur le cathéter comportant le fil de guidage (6).
